# EUROPEAN PATENT APPLICATION

(11) **EP 4 675 260 A1**
(43) Date of publication of application: **07.01.2026**
(21) Application number: 24306093.6
(22) Date of filing: 02.07.2024
(51) Int. Cl.: G01N 21/65

(54) **RAMAN SPECTROSCOPY FOR REAL-TIME MONITORING OF SOLVENT CLEARANCE IN DOWNSTREAM PROCESSES**

(71) Applicant: Merck Patent GmbH, 64293 Darmstadt (DE); Sigma-Aldrich Co. LLC, St. Louis, MO 63103 (US)
(72) Inventor: The designation of the inventor has not yet been filed
(74) Representative: Merck Patent Association

(57) **Abstract**

A method for monitoring solvent clearance in a downstream process includes the steps of providing a calibration model for quantitating concentration of the solvent, collecting Raman spectra in situ during the solvent clearance process, and using the calibration model to quantitate the concentration of solvent. The method is particularly suitable for monitoring solvent clearance in a tangential flow filtration process, such as used in the production of antibody-drug conjugates, lipid nanoparticles and liposomes.

## Description

### BACKGROUND

Organic solvent clearance is a critical and must-have process step for biologic pharmaceutical drugs manufacturing. Tangential flow filtration (TFF) is the most common process to remove organic solvents from drug substance or products. The TFF process typically takes about 2 hours or more and the solvent clearance can only be determined by offline methods. The most commonly used offline methods are chromatographic methods, such as HPLC and GC. Using these methods requires tedious sample preparation to remove the protein and to dilute to range. After the tedious sample preparation, the HPLC run time take an additional 30 minutes.

Solvent clearance in the manufacture of antibody-drug conjugate (ADC) and lipid nanoparticle (LNP) crude products is a critical step to assure their quality and safety. The conventional method is either HPLC or GC based method. And they require protein removal prior the column analysis. Protein removal is tedious and time consuming. And there is potential losing analyte during these procedures. Even more, these conventional methods can only be applied offline. There is no practical real time monitoring method for solvent clearance for ADC or LNP TFF processes. A need exists for such a method.

Raman spectroscopy is a powerful analytical tool used for the identification and characterization of specific compounds within complex mixtures. This non-destructive technique relies on the inelastic scattering of photons, providing detailed information about the vibrational and rotational modes of molecules. By producing unique spectral fingerprints for different compounds, Raman spectroscopy enables accurate identification and quantification of substances of interest.

Monitoring of various components in the manufacture of ADCs with Raman spectroscopy, particularly conjugation starting materials, products, and impurities, such as unconjugated or aggregated mAbs, unconjugated drug, and unconjugated drug-linker has been shown in the art.

U.S. Patent no. 10,563,163 discloses the use of Raman spectroscopy to determine culture parameters, such as glucose, glutamate, ammonia or lactate, in a manufacturing scale bioreactor culture.

U.S. Patent no. 11,274,121 discloses the use of Raman spectroscopy for in-line in an ADC manufacturing process for monitoring concentration of antibody added to the conjugation reaction, unconjugated drug in the retentate, and/or to measure the concentration of free drug impurities in the retentate during a TFF process.

U.S. Patent no. 11,358,984 discloses the use of Raman spectroscopy in a downstream ADC purification process to measure protein concentration and to identify high molecular weight (HMW) impurities, i.e., products-related impurities that contribute to the size heterogeneity of mAb products.

WO2019140141 discloses the use of in-line monitoring, including Raman spectroscopy, to measure analytes in ADC manufacturing processes. Such analytes measured include addition of components added to the ADC conjugation reaction, such as antibody or antigen-binding fragment thereof, a drug, a linker, drug attached to a linker, an antibody or antigen-binding fragment thereof attached to a linker, and/or a conjugation buffer. It also discloses in-line monitoring during a downstream filtration process, to measure concentration of analytes such as unconjugated drug, unconjugated drug-linker, ADC, antibody, antigen-binding fragment, reaction buffer or filtration buffer.

WO20238918 discloses the use of in-line Raman spectroscopy in a perfusion cell culture system to monitor specific biochemical indices including viable cell density (VCD), cell diameter, pH, Pco₂, Po₂, Na⁺ and K⁺ ions, glucose, glutamine, glutamate, lactate, ammonium ions and titer, or osmolality.

While the above references disclose the use of Raman spectroscopy to measure various analytes of interest, none contemplate or suggest the use of Raman spectroscopy to measure solvent removal.

### SUMMARY

Provided are real-time inline methods for monitoring solvent clearance, or removal, during a downstream process.

In one embodiment, a method for monitoring solvent clearance in a downstream process includes the steps of providing a calibration model for quantitating concentration of the solvent, collecting Raman spectra during the solvent clearance process, and using the calibration model to quantitate the concentration of solvent.

In another embodiment, the method provided is an in-line, real-time method for monitoring solvent clearance from a filter retentate in a downstream filtration process comprising the steps of providing a calibration model for quantitating concentration of the solvent, collecting Raman spectra of the filtration retentate in situ, using the calibration model to quantitate the concentration of solvent in the filtrate retentate in real time, and continuously monitoring solvent clearance by repeating steps collecting and quantitating steps until the concentration of solvent in the filtrate retentate is below a predefined level.

Further provided are methods for monitoring trace solvent concentration in a filtration retentate in a downstream process comprising the steps of providing a calibration model for determining concentration of the solvent, collecting Raman spectra during the solvent removal process, and using the calibration model to quantitate the concentration of solvent.

### BRIEF DESCRIPTION OF THE DRAWINGS

Fig. 1 shows the set-up used to illustrate real time online organic solvent clearance monitoring.
Fig. 2 is an illustration of the Raman probe.
Fig. 3 is a diagram showing operation of the Raman analyzer.
Fig. 4 illustrates a system used for offline sampling device for conventional measurements.
Fig. 5 shows an exemplary preprocessed Raman spectra with selected wavelength ranges.

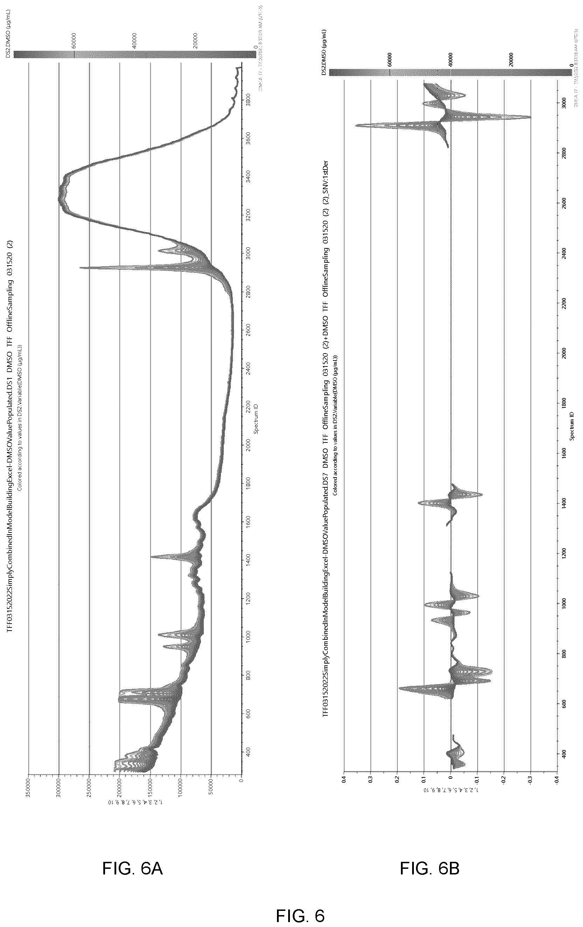

Fig. 6A shows the raw Raman spectra for the training data set. Fig. 6B shows the normalized data within the wavelength ranges of interest.
Fig. 7 shows Table 6, calibration dataset 1, individual standards prepared for Example 1.
Fig. 8 shows Table 7, calibration dataset 2 for Example 1.
Fig. 9 shows a representative chromatogram from HPLC-DAD assay from Example 1.
Fig. 10 shows offline results from HPLC-DAD assay from the first TFF run of Example 1.
Fig. 11 shows offline results from HPLC-DAD assay from the second TFF run of Example 1.
Fig. 12 shows polynomial regression models used in aligning offline and Raman data for the first TFF data set in Example 1.
Fig. 13 shows the alignment of offline HPLC-DAD and polynomial regression models predicted results for the first TFF data set for Example 1.
Fig. 14 shows polynomial regressions used to predict the DMSO concentration at each particular data Raman time point for the second TFF data set of Example 1.
Fig. 15 shows alignment of offline HPLC-DAD and polynomial regression models predicted results at each particular Raman data time point for the second TFF Raman data set from Example 1.
Fig. 16 shows comparison of reference and predicted value from model built using combined standard dataset including the individual standard dataset and standard addition dataset from Example 1.
Fig. 17 shows comparison of reference and predicted value from the model built using the full offline analyses dataset in Example 1.
Fig. 18 shows comparison of reference and predicted value from the model built using all three data sets with reduced concentration range (1 to 599 µg/mL) in Example 1.
Fig. 19 shows an exemplary chromatogram of the HS-GS-FID assay obtained when building calibration dataset 3 of Example 2.
Fig. 20 shows the offline results from the HS-GS-FID assay for first and second TFF from Example 2.
Fig. 21 shows the polynomial regression models used to predict the DMSO concentration at each particular Raman data point from offline HS-GC-FID results for TFF-modeling building data set in Example 2.
Fig. 22 shows alignment of offline HS-GC-FID and Raman polynomial regression models predicted results for ethanol clearance by TFF in Example 2.
Fig. 23 shows five different polynomial regression models applied when aligned offline HS-GC-FID and Raman data for TFF-monitoring dataset in Example 2.
Fig. 24 shows the alignment of offline HS-GC-FID and Raman polynomial regression models predicted results at each particular data time point in TFF-monitoring dataset in Example 2.
Fig. 25 shows the comparison of reference and predicted value from model built using combined standard dataset including individual standard and standard addition dataset of Example 2.
Fig. 26 shows the comparison of reference and predicted value from model built with the full offline analysis dataset in Example 2.
Fig. 27 shows the comparison of reference and predicted value from model built using a reduced offline analysis dataset (only included data within 1 to 1000 µg/mL range) in Example 2.

### DETAILED DESCRIPTION

Solvent clearance in the manufacture of antibody-drug conjugate (ADC) crude products is a critical step to assure their quality and safety. The conventional method is either HPLC or GC based method. And they require protein removal prior the column analysis. Protein removal is tedious and time consuming. And there is potential losing analyte during these procedures. Even more, these conventional methods can only be applied offline.

Applying Raman spectroscopy to monitor the solvent clearance in TFF processes significantly overcomes all the downfalls associated with traditional offline analysis approaches. Probe-based Raman analyzers facilitate real time inline analysis of solvent clearance for TFF process with very fast turnover time, approximately 3 minutes or less per sampling. Further, no sample preparation, such as protein separation and dilution are required. Combining in-line Raman spectroscopy with a partial least squares-based modeling and prediction approach for quantification provides very robust performance since it utilizes multiple ranges of featured Raman shifts instead of a single frequency shift. The high sampling frequency, robustness and inline feature make probe-based Raman spectroscopy a compelling approach for real-time solvent clearance monitoring for TFF processes.

Use of Raman spectroscopy in downstream processes offers several advantages, including real-time in-line analysis, no need for sample preparation, no sample consumption, high sampling frequency, and less or no disruption to processes.

To illustrate the methods described herein, a ProCellics^{™} Raman analyzer with Bio4C^{®} PAT Raman software (MilliporeSigma, Burlington, MA) was used. It is noted that other Raman systems may be used as well.

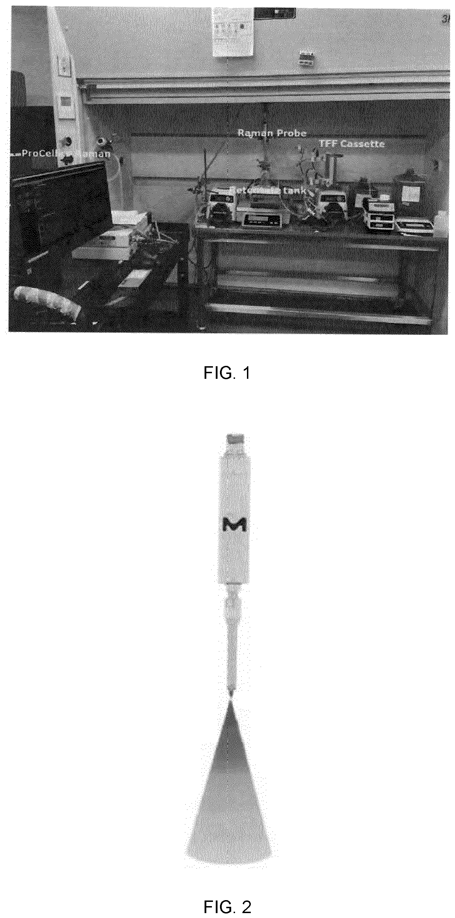

Fig. 1 illustrates the actual set up of a real time online organic solvent clearance monitoring by the probe-based Raman analyzer. The TFF system includes a digital balance, peristaltic pump and flow/pressure sensor and was set up as illustrated in Fig. 1. Equipment list and typical parameters are listed in Table 1 below. One skilled in the art would understand that the equipment and/or specific parameters may be varied, but still work with the methods provided herein.

**Table 1**

| **Equipment** | **Experiment parameters** |
|---|---|
| Q30 pump head single use | Pump feed flowrate: 4-6 L/min per m² |
| Masterflex^{®} L/S pump (two) | mass load: <5 grams |
| Pellicon^{®} 3 Ultracel^{®} 30 kDa C screen (MilliporeSigma, Burlington, MA) | Membrane loading: <200 g/m² |
| 3 x 88 cm² membranes into Pellicon^{®} mini cassette holder | TMP 10~20 psi |
| | 12 DVs |

A laser (785nm, 500 mw, illustrated in Fig. 2) emits through an optical fiber connected to a probe head/probe tube which is immersed into the process being analyzing. Briefly, the laser excites the different molecules/chemical bonds within the analyzed material, shifting the frequency of light, with the shift corresponding to specific molecular vibrations. This Raman signal is collected back through the fiber optic probe and sent to the analyzer where a charge-coupled device (CCD) collects the photons.

The acquired spectra are then communicated to the Bio4C^{®} PAT Raman software and this spectra corresponds to a unique chemical fingerprint of the analyzed material. Then, the software transforms the acquired Raman spectral fingerprint into critical quality attributes (CQAs) & critical control points (CCPs) process values in real-time.

The system used in this illustration is compliant with European union requirements of EMC 2014/30/UE (EN 55011:2009+A1/2010 class A, EN 61326-1:2013) and with 2014/35/EU low voltage directive; the software is CFR 21 Part 11 compliant for GMP implementation, and the software supports OPC-UA communication protocol.

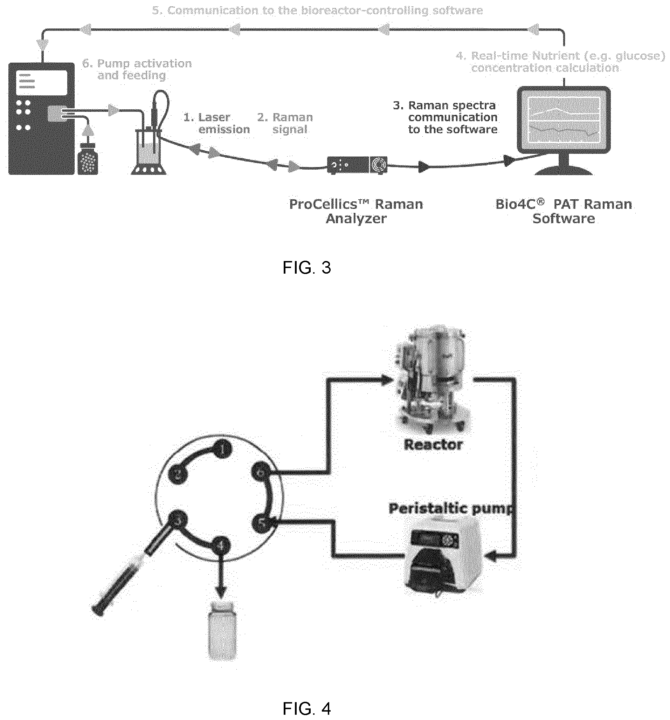

The use of the Raman system is shown schematically in Fig. 3. The ProCellics^{™} Raman analyzer is preferably operated in single channel mode to achieve the best sensitivity. Integration time and number of spectra averaged are chosen to balance the acquisition time with the quality of the spectra obtained. In a preferred method, a 30 second integration time is used, and 5 spectra are averaged to obtain each data point. Longer integration times and/or increasing the number of spectra averaged will increase sensitivity, though at the cost of longer analysis times. In other embodiments, the number of spectra obtained could be varied from one to many.

A training data set can be collected from a standard sample set and/or a standard addition sample set with projected concentration value. A training model can also be built from a standard data set that collected in model building mode from an actual TFF run with concentration values obtained from offline analyses. An online sampling device, illustrated in Fig. 4 is used to collect offline sample at given times. The offline samples are analyzed by a HPLC-DAD method with offline protein separation and sample dilution.

The offline sampling (shown in Fig. 4) is accomplished by using a system including peristaltic pump and a six-port two-position sample switch valve. The peristaltic pump is operated at 4 mL/minute to circulate the retentate through the valve. Sample valve is switched to the load position (2) for 5 s to collect ~0.5 mL sample into a sample vial. Sample collection frequency is every 5 minutes.

The offline sampling system as shown in Fig. 4 has 6 ports, labeled 1-6. In position 2, ports 1-6, 4-5, and 2-3 are connected. Sample is collected to the vial. Sample volume is determined by time and flow rate. In position 1, ports 1-2, 3-4, and 5-6 are connected. 1. Sample is circulated back to the reactor. 2. Using the syringe filled with air, residual sample is pushed to the collection vial. 3. Solvent can be used to clean the ports and sample collection tubing prior to next use.

The collected offline samples are analyzed by conventional HPLC-DAD method. After initial dilution, spin filter is used to separate the protein or other large molecules to remove matrix effect. Flow through is collected and further diluted to range according to its projected concentration. The diluted samples are then injected on mix mode column and concentration is calculated against the standard. A calculated concertation at each Raman sampling time points is obtained from polynomial regression models that established on offline results and offline sampling time.

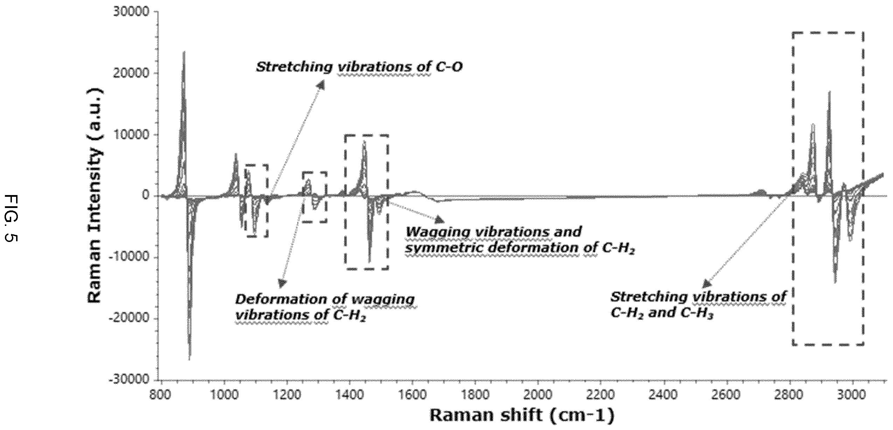

A typical partial least squares (PLS) model is built with collected Raman spectra and calculated solvent concentration from the offline results. Two data preprocessing steps are applied, one is standard normal variate, the other is Savitzky-Golay derivative. While the whole Raman spectrum could be used in model building, using selected shift ranges based on the Raman spectrum the solvent of interest is preferred. For the solvent DMSO, for example, the selected Raman shift ranges 20-500, 600-1150, 1290- 1500, and 2800-3100 cm⁻¹ may be used. For other solvents, such as dimethylacetamide (DMA), isopropyl alcohol (IPA), or others used in ADC, LNP or liposome production and downstream processing, other Raman shift ranges will be chosen based on the spectrum of the particular solvent. Fig. 5 shows an exemplary preprocessed Raman spectra with selected wavelength ranges for DMSO.

In one run performed, a first PLS model built with the full offline samples analysis dataset provided very high prediction accuracy for concentration range from 500 µg/mL to 6% (Table 2), while its prediction accuracy for concentration range below 500 µg/mL was poor, as shown below the heavy line. To meet the requirement of ICH Q3C(R8) for typical API concentration at 20 mg/mL. A quantification limit as low as 100 µg/mL is needed.

**Table 2**

| TFF time (min.) | Reference value (µg/mL) | Model predicted value (µg/mL) | Recovery (%) |
|---|---|---|---|
| 0.0 | 66087.0 | 65239 | 99 |
| 2.0 | 54696.9 | 56128 | 103 |
| 4.7 | 42257.4 | 46193.2 | 109 |
| 7.5 | 33225.8 | 35997.8 | 108 |
| 10.2 | 26742.5 | 28010.9 | 105 |
| 13.0 | 21948.0 | 20875.3 | 95 |
| 15.7 | 17982.6 | 15932.7 | 89 |
| 18.5 | 13986.9 | 12600.3 | 90 |
| 21.2 | 9101.3 | 9985 | 109 |
| 24.0 | 7188.9 | 7780.66 | 108 |
| 26.7 | 5728.7 | 6091.33 | 106 |
| 29.5 | 4502.7 | 4745.79 | 105 |
| 32.2 | 3492.9 | 3705.6 | 106 |
| 35.0 | 2681.3 | 2879.52 | 107 |
| 37.7 | 2049.8 | 2232.78 | 109 |
| 40.5 | 1580.3 | 1639.75 | 104 |
| 43.2 | 1254.8 | 1279.8 | 102 |
| 46.0 | 1055.4 | 1033.34 | 98 |
| 48.7 | 785.2 | 812.758 | 104 |
| 51.5 | 605.3 | 629.211 | 104 |
| 54.2 | 468.3 | 432.964 | 92 |
| 57.0 | 366.5 | 180.815 | 49 |
| 59.8 | 292.4 | 259.776 | 89 |
| 62.5 | 238.5 | 168.883 | 71 |
| 65.3 | 197.1 | 163.94 | 83 |
| 68.0 | 160.8 | 124.25 | 77 |
| 70.8 | 122.0 | 75.3872 | 62 |
| 73.5 | 91.8 | 27.8481 | 30 |
| 76.3 | 78.0 | 59.7988 | 77 |
| 79.0 | 65.7 | 18.915 | 29 |

The model building algorithm was further optimized with a standard data set that only contains the low concentration data, and new auto scale approach. The optimized model provided very good quantification accuracy for concentration range below 8000 µg/mL. Table 3 presents an example quantification results and calculated recovery. The quantification limit of the optimized model appears to be around 30 µg/mL such a quantitative sensitivity makes the application of Raman for online monitoring solvent clearance practical for ADC processes.

**Table 3**

| TFF time (min.) | Reference value (µg/mL) | Model predicted value (µg/mL) | Recovery (%) |
|---|---|---|---|
| 0.0 | 66087.0 | 23499.6 | 36.0 |
| 2.0 | 54696.9 | 17639.3 | 32.0 |
| 4.7 | 42257.4 | 15477.3 | 37.0 |
| 7.5 | 33225.8 | 15956.6 | 48.0 |
| 10.2 | 26742.5 | 15264.6 | 57.0 |
| 13.0 | 21948.0 | 14856.8 | 68.0 |
| 15.7 | 17982.6 | 12820.3 | 71.0 |
| 18.5 | 13986.9 | 10225.9 | 73.0 |
| 21.2 | 9101.3 | 7950.9 | 87.0 |
| 24.0 | 7189.0 | 6189.9 | 86.0 |
| 26.7 | 5728.7 | 4829.9 | 84.0 |
| 29.5 | 4205.7 | 3765.6 | 84.0 |
| 32.2 | 3492.9 | 2915.2 | 83.0 |
| 35.0 | 2681.3 | 2282.3 | 85.0 |
| 37.7 | 2049.8 | 1779.0 | 87.0 |
| 40.5 | 1580.3 | 1435.4 | 91.0 |
| 43.2 | 1254.9 | 1082.1 | 86.0 |
| 46.0 | 1055.4 | 861.0 | 82.0 |
| 48.7 | 785.2 | 685.4 | 87.0 |
| 51.5 | 605.3 | 531.7 | 88.0 |
| 54.2 | 468.3 | 432.4 | 92.0 |
| 57.0 | 366.5 | 348.3 | 95.0 |
| 59.8 | 292.4 | 263.0 | 90.0 |
| 62.5 | 238.5 | 214.8 | 90 |
| 65.3 | 197.1 | 181.0 | 92 |
| 68.0 | 160.8 | 142.7 | 89 |
| 70.8 | 122.0 | 116.2 | 95 |
| 73.5 | 91.8 | 95.2 | 104 |
| 76.3 | 78.0 | 82.3 | 106 |
| 79.0 | 65.7 | 54.6 | 83 |
| 81.8 | 55.1 | 45.7 | 88 |
| 84.5 | 45.9 | 41.6 | 91 |
| 87.3 | 38.3 | 39.0 | 102 |
| 90.0 | 32.3 | 36.3 | 112 |
| 92.8 | 27.8 | 38.4 | 138 |
| 95.5 | 24.9 | 21.6 | 87 |
| 98.3 | 20.6 | 25.4 | 123 |
| 101.0 | 18.5 | 68.0 | 366 |
| 103.8 | 16.6 | 24.4 | 147 |
| 106.5 | 14.9 | 21.9 | 146 |
| 109.3 | 13.4 | 44.4 | 331 |

Provided are real-time inline methods for monitoring solvent clearance, or removal, during a downstream process, such as a filtration or column chromatography process, during the production of drugs, such as ADCs, lipid nanoparticles (LNPs) or liposomes, though the method is suitable for any downstream solvent removal process. The methods use Raman spectroscopy and multivariate analysis monitor and/or quantitate the trace amounts of solvent during the removal process. Advantageously, a fiber optic probe is used directly in the retentate, eliminating the need to remove samples for offline analysis.

Provided are methods for monitoring solvent clearance in a downstream process; these models include the steps of (a) providing a calibration model for quantitating concentration of the solvent, (b) collecting Raman spectra during the solvent clearance process, and (c) using the calibration model to quantitate the concentration of solvent. In some embodiments, the monitoring of solvent clearance is done in real-time. In some embodiments, the Raman spectra are collected in situ, such as with a fiber optic probe. In other embodiments, samples may be collected and analyzed offline.

In various embodiments, steps (b) and (c) are repeated until the concentration of solvent is below a pre-defined level. In some embodiments, the pre-defined level of solvent concentration corresponds to the international council for harmonisation (ICH) guideline for residual solvents Q3C(R8) for that solvent.

In various embodiments, the calibration model may be any of a linear regression model, a principal components regression (PCR) model, a partial least squares (PLS) regression model, a support vector machines (SCM) model, a neural network model, and an artificial intelligence (AI) model. Other models may also be used. In a preferred embodiment, the calibration model is a PLS regression model.

In various embodiments, the downstream process is a tangential flow filtration (TFF) process or a column purification process. In some embodiments, the downstream process is a downstream antibody-drug conjugate (ADC) production process. When the downstream process is an ADC process, exemplary solvents include, but are not limited to dimethyl sulfoxide (DMSO), dimethylacetamide (DMA), and isopropyl alcohol (IPA). The methods described herein are not limited to particular solvents and are suitable for any solvents used in downstream processes.

In some embodiments, the downstream process is a downstream lipid nanoparticle (LNP) or liposome production process. In such embodiments, an exemplary solvent is ethanol, though the methods described herein are not limited to ethanol or other particular solvents.

Also provided is an in-line, real-time method for monitoring solvent clearance from a filter retentate in a downstream filtration process including the steps of providing a regression model for quantitating concentration of the solvent, collecting Raman spectra of the filtration retentate in situ, using the regression model to quantitate the concentration of solvent in the filtrate retentate in real time, and continuously monitoring solvent clearance by repeating the collecting Raman spectra and using the regression model to quantitate the concentration of solvent steps until the concentration of solvent in the filtrate retentate is below a predefined level. In some embodiments, the filtration process is a TFF process.

The regression model used with this method may be selected from a linear regression model, a principal components regression (PCR) model, a partial least squares (PLS) regression model, a support vector machines (SCM) model, a neural network model, or an artificial intelligence (AI) model, or other models. In one preferred embodiment, the regression model is a PLS regression model.

In some embodiments, the predetermined level of solvent concentration corresponds to the international council for harmonisation (ICH) guideline for residual solvents Q3C(R8) for that solvent.

Further provided is a method for monitoring trace solvent concentration in a filtration retentate in a downstream process including the steps of (a) providing a calibration model for determining concentration of the solvent, (b) collecting Raman spectra during the solvent removal process, and (c) using the calibration model to quantitate the concentration of solvent.

In various embodiments, the downstream process is a tangential flow filtration (TFF) process or a column chromatography process.

In various embodiments, exemplary calibration models may be, but are not limited to linear regression models, principal components regression (PCR) models, partial least squares (PLS) regression models, support vector machines (SCM) models, neural network models, or artificial intelligence (AI) models.

In some embodiments, the monitoring is done in real-time. In some embodiments, the monitoring is done in situ. In some embodiments, steps b and c are repeated until the trace solvent concentration is below a pre-defined level. In such embodiments, the pre-defined level of trace solvent concentration may correspond to, e.g., the international council for Harmonisation (ICH) Guideline for Residual Solvents Q3C(R8) for that solvent.

In some embodiments, the downstream process is a downstream antibody-drug conjugate (ADC) production process. In various embodiments, exemplary solvents may include, but are not limited to DMSO, DMA and IPA.

In other embodiments, the downstream process is a downstream lipid nanoparticle (LNP) or liposome production process. In such embodiments, the solvent may be, e.g., ethanol.

Further provided is a method for quantitating residual solvent in a downstream process including the steps of providing a calibration model for quantitating concentration of the solvent, collecting Raman spectra of a filter retentate during the downstream process, and using the calibration model to quantitate the concentration of residual solvent in the filter retentate.

In various embodiments, the calibration model may be selected from a linear regression model, a principal components regression (PCR) model, a partial least squares (PLS) regression model, a support vector machines (SCM) model, a neural network model, or an artificial intelligence (AI) model. In some embodiments, the calibration model is a PLS regression model.

In various embodiments, the downstream process may be a tangential flow filtration (TFF) process or a column purification process.

In some embodiments, the quantitation of residual solvent is done in real-time. In some embodiments, the Raman spectra are collected in situ.

In preferred embodiments, steps b and c are repeated until the residual solvent concentration is below a pre-defined level. Such pre-defined level of residual solvent may correspond to the international council for harmonisation (ICH) guideline for residual solvents Q3C(R8) for that solvent.

In some embodiments, the downstream process is a downstream antibody-drug conjugate (ADC) production process. In such embodiments, exemplary solvents may include, but are not limited to DMSO, DMA, IPA and combinations thereof. In other embodiments, downstream process is a downstream lipid nanoparticle (LNP) of liposome production process. In such embodiments, an exemplary solvent is ethanol.

Further provided is a method for measuring solvent clearance in a downstream process including the steps of providing a calibration model for quantitating concentration of the solvent, collecting Raman spectra during the solvent clearance process, and using the calibration model to quantitate the concentration of solvent.

Exemplary calibration models useful in this method include linear regression models, principal components regression (PCR) models, partial least squares (PLS) regression models, support vector machines (SCM) models, neural network models, and artificial intelligence (AI) models.

In various embodiments, the downstream process may be, for example, TFF or column chromatography.

### EXAMPLES

### Example 1. Real time inline monitoring of DMSO clearance by TFF

Online monitoring was performed using a ProCellics^{™} Raman analyzer. The offline sampling system described above facilitated collection of sample at different time points for comparison using conventional methods without disrupting the process. The experimental parameters are shown in Table 4.

**Table 4**

| **Equipment** | **Experiment parameters** |
|---|---|
| Q30 pump head single use | Pump feed flowrate ~ 140 mL/min |
| Masterflex^{®} L/S pump (two) | Protein load ~ 3.6 grams |
| Pellicon^{®} 3 Ultracel^{®} 30 kDa C screen | Membrane loading ~ 136.5 g/m² |
| 3 x 88 cm² membranes into Pellicon^{®} mini cassette holder | TMP-14 psi |
| | 12 DVs |

For maximizing the sensitivity, single channel was used to monitor the retentate only. Data acquisition: 30 s integration time was used, averaging of 5 spectra for each data point.

### Experiment batches:

Data for first TFF were collected in model building mode as standard data set. Data for second TFF were collected in monitoring mode. Model built from standard data sets was used for online monitoring.

### Offline sampling procedures:

The offline sampling system described above was used to collect samples for offline measurements. The peristaltic pump was operated at 4 mL/minute to circulate the retentate. The sample valve was switched to the load position (2) for 8s to collect a 0.5 mL sample. Sample collection frequency was every 5 minutes during the experiment.

### Calibration dataset 1

Calibration dataset 1 was built using individual standards. The ProCellics^{™} Raman analyzer acquisition parameters were optimized with 0.1 % DMSO using 30s integration time, 5 spectra average. In batch collection mode, 1.5 minutes waiting time was set to allow switching samples or standard addition.

Three stock standard solution was prepared by weighing out designated DMSO and dissolving them in conjugation buffer (100 mM histidine, 50 mM NaCl, pH 6.8) as shown in Table 5.

**Table 5**

| Standard | Weight of DMSO (g) |
|---|---|
| Std-10 DMSO | 10.9060 |
| Std-7.5 DMSO | 7.6948 |
| Std-5 DMSO | 5.0506 |
| Std-2.5 DMSO | 2.5365 |

### Calibration dataset 1- individual standards, standard set preparation:

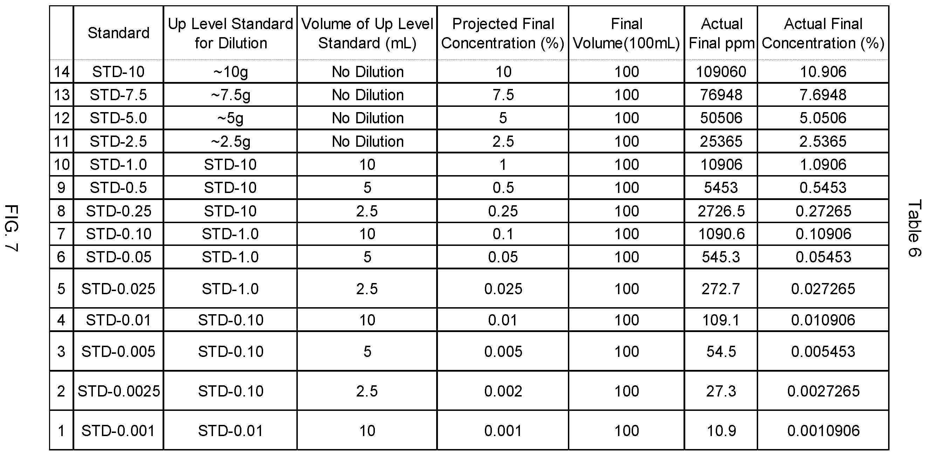

Conjugation buffer (100 mM histidine 50 mM NaCl, pH 6.8) was used as matrix with ~ 700 mL of crude ADC produced was used (for better matrix). Protein concentration is 9 mg/mL, and DMSO concentration is 7.5%. Different standards were prepared according to the standard set in Table 6 (Fig. 7).

### Calibration dataset 2 - standard addition standards, set preparation:

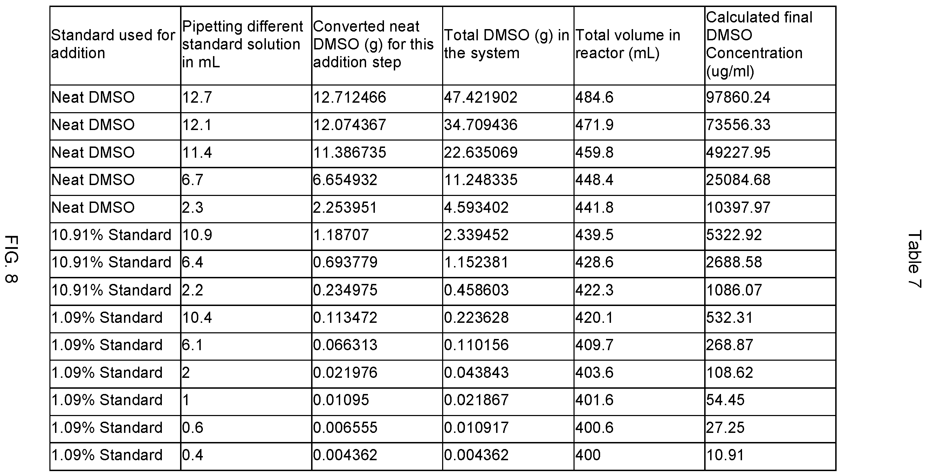

Two standards (1.09% and 10.91%) from the individual standard set and neat DMSO were used for the standard DMSO addition experiment. TFF was set up as designated ADC process just without the membrane. Q30 pump feed flow at ~100 mL/min., Overhead mixer set to 60 rpm, standard addition was accomplished according to Table 7 (Fig. 8).

The offline HPLC-DAD method overview is shown in Table 8 below:

**Table 8**

| | | |
|---|---|---|
| Column: | Supelcogel C-610H column, 300 x 7.8 mm, Sigma-Aldrich P/N 59320-U | |
| Mobile phase: | 0.1% phosphoric acid in purified water | |
| Instrument parameters: | Flow rate: | 0.7 mL/min |
| | Injection volume: | 100 µl |
| | Acquisition time | 30 minutes |
| | UV wavelength | 210 nm |
| | Column compartment temp. | 35 ± 1°c |
| Sample preparation: | Amicon^{®} Ultra-2 centrifugal filter unit Ultracel^{®} 100 regenerated cellulose membrane | |

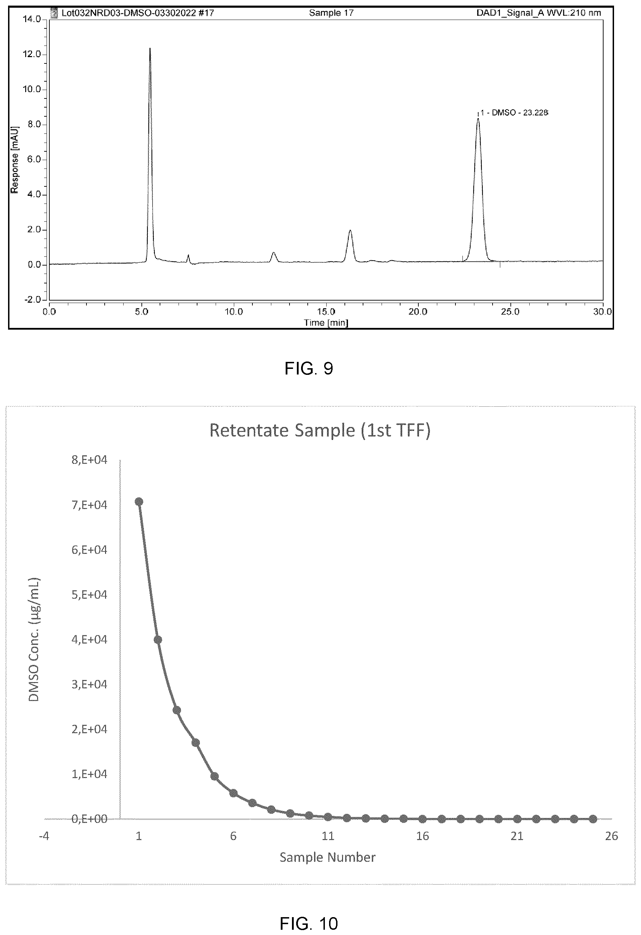

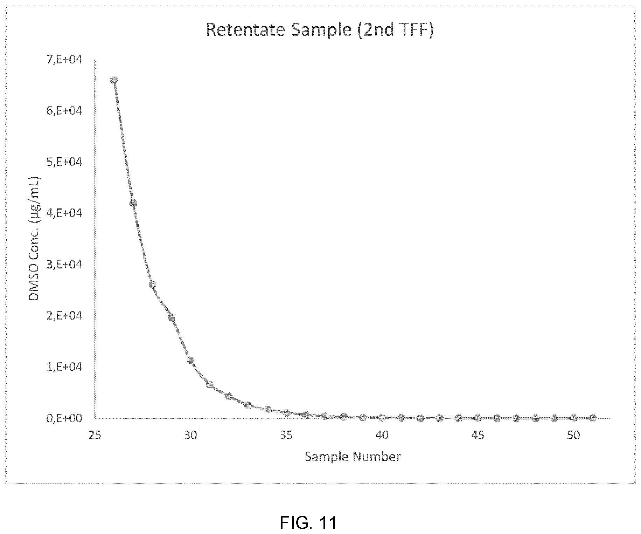

An exemplary chromatogram is shown in Fig. 9. Figs. 10 and 11 show the offline results from the HPLC-DAD assay for the first TFF samples collected data for model building (Fig. 10) and the second TFF real time monitoring (Fig. 11).

Calibration dataset 3-aligning the offline HPLC-DAD and Raman data point.

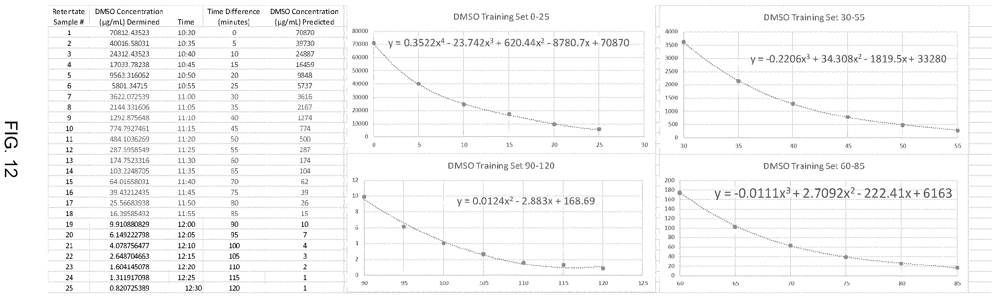

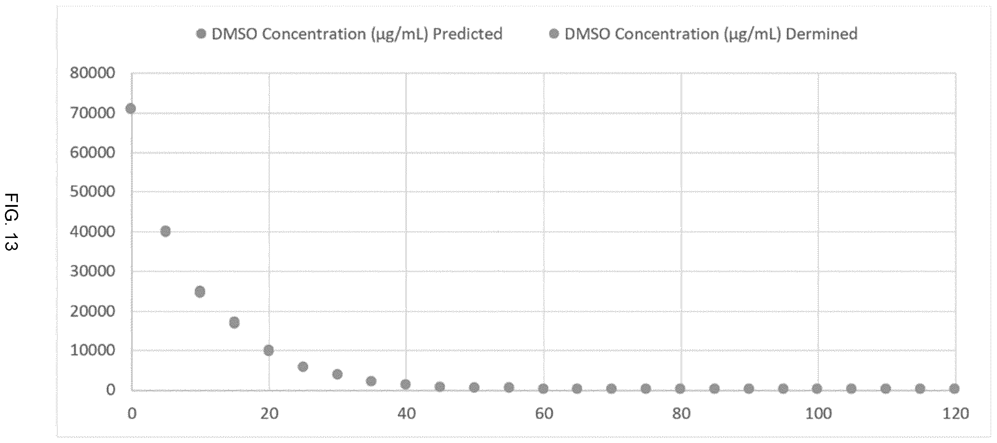

Offline analysis was sampling at every 5 minutes. Raman sampling was done every 2-3/4 minutes. Polynomial regressions were used to predict the DMSO concentration at each time point Raman spectrum were recorded. Due to the very broad dynamic range, four different polynomial regression models were applied to different sections to assure the prediction accuracy, as shown in Fig. 12. The comparison of determined and predicted DMSO concentration value is shown in Fig. 13.

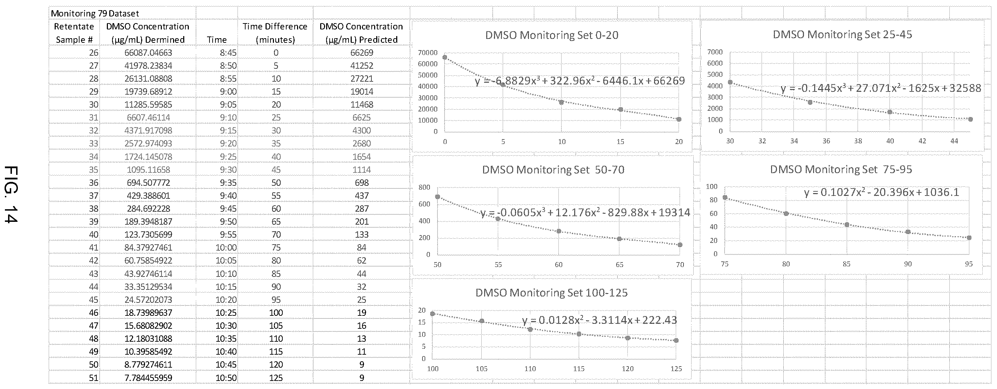

Fig. 14 shows polynomial regressions used to predict the DMSO concentration at each time point Raman spectra were recorded. Due to the very broad dynamic range, five different polynomial regression models were applied to different sections to assure the prediction accuracy.

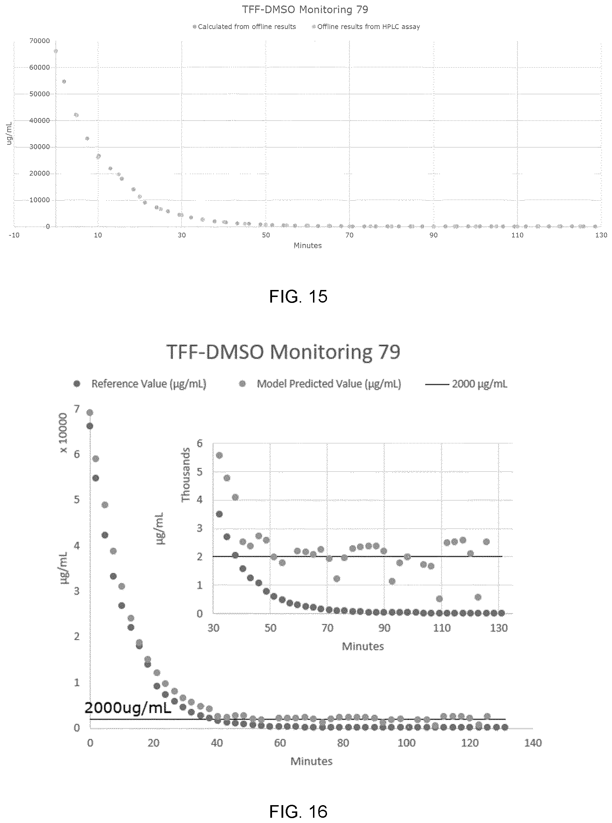

Fig. 15 shows alignment of offline HPLC-DAD and polynomial regression predicted results for Raman data for solvent clearance over 130 minutes.

Next, a comparative model was built from combined standard data sets. Data preprocessing parameters: SNV (standard normal variate) (with "water" as type selection within the Bio4C^{®} PAT Raman software), SG (Savitzky-Golay)-derivative, selected Raman ranges: 320-500, 600-1150, 1290-1500, and 2800-3100 cm⁻¹. The calibration dataset was built using the individual standard dataset and standard addition dataset, but without the offline sample analysis dataset. The resulting calibration curve is shown in FIG. 16, and the data is shown in Table 9, below.

**Table 9**

| TFF time(minutes) | Reference value (µg/mL) | Model predicted value (µg/mL) | Recovery (%) |
|---|---|---|---|
| 0.0 | 66087.0 | 69149.6 | 105 |
| 2.0 | 54696.9 | 59063.4 | 108 |
| 4.7 | 42257.4 | 48908.8 | 116 |
| 7.5 | 33225.8 | 38820.2 | 117 |
| 10.2 | 26742.5 | 30961.6 | 116 |
| 13.0 | 21948.0 | 24027.9 | 109 |
| 15.7 | 17982.6 | 18749.1 | 104 |
| 18.5 | 13986.9 | 15021.9 | 107 |
| 21.2 | 9101.3 | 12001.7 | 132 |
| 24.0 | 7188.9 | 9737.06 | 135 |
| 26.7 | 5728.7 | 7972.38 | 139 |
| 29.5 | 4502.7 | 6536.14 | 145 |
| 32.2 | 3492.9 | 5567.49 | 159 |
| 35.0 | 2681.3 | 4777.67 | 178 |
| 37.7 | 2049.8 | 4097.43 | 200 |
| 40.5 | 1580.3 | 2503 | 158 |
| 43.2 | 1254.8 | 2369.94 | 189 |
| 46.0 | 1055.4 | 2736.8 | 259 |
| 48.7 | 785.2 | 2589.7 | 330 |
| 51.5 | 605.3 | 1974.63 | 326 |
| 54.2 | 468.3 | 1764.47 | 377 |
| 57.0 | 366.5 | -359.353 | -98 |
| 59.8 | 292.4 | 2202.64 | 753 |
| 62.5 | 238.5 | 2150.84 | 902 |
| 65.3 | 197.1 | 2078.56 | 1054 |
| 68.0 | 160.8 | 2260.4 | 1406 |
| 70.8 | 122.0 | 1926.37 | 1579 |
| 73.5 | 91.8 | 1224.07 | 1334 |
| 76.3 | 78.0 | 1943.2 | 2492 |
| 79.0 | 65.7 | 2277.57 | 3464 |

As shown in FIG. 16 and Table 9, the model predicted values exhibited a large deviation from reference values, particularly in the low concentration range. The model was found to have a detection limit around 2000-3500 µg/mL.

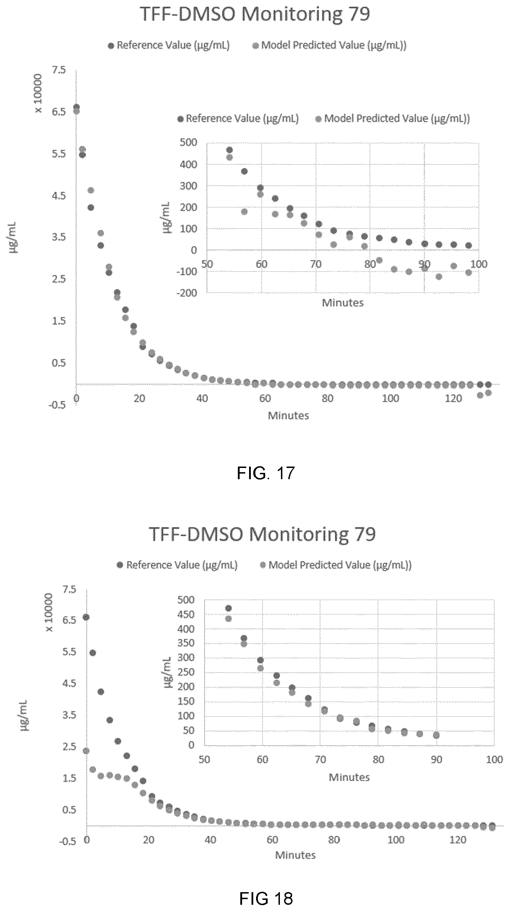

The comparative model was followed by building a model using the offline analyses dataset. The data processing parameters are the same as for the comparative model above. Only the offline sample analysis dataset was used to build this model. The calibration curve is shown in FIG. 17. The data is shown in Table 10, below.

**Table 10**

| TFF time(minutes) | Reference value (µg/mL) | Model predicted value (µg/mL) | Recovery (%) |
|---|---|---|---|
| 0.0 | 66087.0 | 65239 | 99 |
| 2.0 | 54696.9 | 56128 | 103 |
| 4.7 | 42257.4 | 46193.2 | 109 |
| 7.5 | 33225.8 | 35997.8 | 108 |
| 10.2 | 26742.5 | 28010.9 | 105 |
| 13.0 | 21948.0 | 20875.3 | 95 |
| 15.7 | 17982.6 | 15932.7 | 89 |
| 18.5 | 13986.9 | 12600.3 | 90 |
| 21.2 | 9101.3 | 9885 | 109 |
| 24.0 | 7188.9 | 7780.66 | 108 |
| 26.7 | 5728.7 | 6091.33 | 106 |
| 29.5 | 4502.7 | 4745.79 | 105 |
| 32.2 | 3492.9 | 3705.6 | 106 |
| 35.0 | 2681.3 | 2879.52 | 107 |
| 37.7 | 2049.8 | 2232.78 | 109 |
| 40.5 | 1580.3 | 1639.75 | 104 |
| 43.2 | 1254.8 | 1279.8 | 102 |
| 46.0 | 1055.4 | 1033.34 | 98 |
| 48.7 | 785.2 | 812.758 | 104 |
| 51.5 | 605.3 | 629.211 | 104 |
| 54.2 | 468.3 | 432.964 | 92 |
| 57.0 | 366.5 | 180.815 | 49 |
| 59.8 | 292.4 | 259.776 | 89 |
| 62.5 | 238.5 | 168.883 | 71 |
| 65.3 | 197.1 | 163.94 | 83 |
| 68.0 | 160.8 | 124.25 | 77 |
| 70.8 | 122.0 | 75.3872 | 62 |
| 73.5 | 91.8 | 27.8481 | 30 |
| 76.3 | 78.0 | 59.7988 | 77 |
| 79.0 | 65.7 | 18.915 | 29 |

The predicted values are very comparable from reference value. Compared with the previous model, this model has significantly improved detection limit. However, the prediction accuracy below 500 µg/mL is still poor.

Next a model was built using combined individual standard dataset, the standard addition dataset, and the reduced offline samples analysis dataset. The same model building parameters are used above. The comparison of reference and model predicted value is shown in FIG. 18, and the data is in Table 11.

**Table 11**

| TFF time(minutes) | Reference value (µg/mL) | Model predicted value (µg/mL) | Recovery (%) |
|---|---|---|---|
| 0.0 | 66087.0 | 23499.5 | 36 |
| 2.0 | 54696.9 | 17639.3 | 32 |
| 4.7 | 42257.4 | 15477.3 | 37 |
| 7.5 | 33225.8 | 15956.6 | 48 |
| 10.2 | 26742.5 | 15264.6 | 57 |
| 13.0 | 21948.0 | 14856.8 | 68 |
| 15.7 | 17982.6 | 12820.3 | 71 |
| 18.5 | 13986.9 | 10225.9 | 73 |
| 21.2 | 9101.3 | 7950.9 | 87 |
| 24.0 | 7189.0 | 6189.9 | 86 |
| 26.7 | 5728.7 | 4829.9 | 84 |
| 29.5 | 4502.7 | 3765.6 | 84 |
| 32.2 | 3492.9 | 2915.2 | 83 |
| 35.0 | 2681.3 | 2282.3 | 85 |
| 37.7 | 2049.8 | 1779.0 | 87 |
| 40.5 | 1580.3 | 1435.4 | 91 |
| 43.2 | 1254.9 | 1082.1 | 86 |
| 46.0 | 1055.4 | 861.0 | 82 |
| 48.7 | 785.2 | 685.4 | 87 |
| 51.5 | 605.3 | 531.7 | 88 |
| 54.2 | 468.3 | 432.4 | 92 |
| 57.0 | 366.5 | 348.3 | 95 |
| 59.8 | 292.4 | 263.0 | 90 |
| 62.5 | 238.5 | 214.8 | 90 |
| 65.3 | 197.1 | 181.0 | 92 |
| 68.0 | 160.8 | 142.7 | 89 |
| 70.8 | 122.0 | 116.2 | 95 |
| 73.5 | 91.8 | 95.2 | 104 |
| 76.3 | 78.0 | 82.3 | 106 |
| 79.0 | 65.7 | 54.6 | 83 |
| 81.8 | 55.1 | 48.7 | 88 |
| 84.5 | 45.9 | 41.6 | 91 |
| 87.3 | 38.3 | 39.0 | 102 |
| 90.0 | 32.3 | 36.3 | 112 |
| 92.8 | 27.8 | 38.4 | 138 |
| 95.5 | 24.9 | 21.6 | 87 |
| 98.3 | 20.6 | 25.4 | 123 |
| 101.0 | 18.5 | 68.0 | 366 |
| 103.8 | 16.6 | 24.4 | 147 |
| 106.5 | 14.9 | 21.9 | 146 |
| 109.3 | 13.4 | 44.4 | 331 |

Using the combination of all three datasets provided poor predication accuracy in high concentration range, though the accuracy gets better starting below 10,000 µg/mL the high concentration range results are less important than the low concentration range, which the ICH limit fell in. The limitation of the model appears to be around 30 µg/mL, which is below the ICH limit 100 µg/mL (5000 ppm for 20 mg/mL API).

### Example 2. Real time inline monitoring of ethanol clearance by TFF

Real time inline monitoring of ethanol clearance in TFF by Raman spectroscopy in a lipid nanoparticle (LNP) manufacture process was studied. The experiment setup for TFF is shown in Table 12, below.

**Table 12**

| **Equipment** | **Experiment parameters** |
|---|---|
| Q30 pump head single use | Pump feed flowrate ~ 140 mL/min |
| Masterflex^{®} L/S pump (two) | LNP load ~ 2.4 grams |
| Pellicon^{®} 3 Ultracel^{®} 30 kDa C screen | Membrane loading ~ 91 g/m² |
| 3 x 88 cm² membranes into Pellicon^{®} mini cassette holder | TMP~10 psi 12dvs |

A ProCellics^{™} Raman analyzer was used. For maximizing the sensitivity, single channel was used to monitor the retentate only. Data acquisition: 30s integration time was used, averaging of 5 spectra for each data point.

Experiment batches: data for first TFF were collected in model building mode. Data for second TFF were collected in monitoring mode. Model built from standard data sets was used for online monitoring.

For offline sampling, the sampling system detailed above and in example 1 was used. Offline sampling procedures: peristaltic pump was operated at 4 mL/minute to circulate the retentate, sample valve was switched to the load position (2) for 8s to collect 0.5 mL sample, sample collection frequency was every 5 minutes.

For the calibration dataset, the ProCellics^{™} instrument acquisition parameters were optimized with 0.5% ethanol. Data were acquired with 30s integration time, 5 spectra average. in batch collection mode, 1.5 minutes waiting time was set to allow switching samples or standard addition.

### Preparation of the calibration standard set:

Filtered final LNP sample from a large-scale production was used as matrix for standard preparation. (5mM:5mM DSPC:cholesterol liposome with 0.0017% residual ethanol). Different standards were prepared according to the standard set table, Table 13, below.

**Table 13**

| Standard name | Ethanol % | Pure ethanol volume, mL | Filtered liposome sample vol., mL | Total volume, mL |
|---|---|---|---|---|
| Std 10 | 10 | 3.000 | 27.00 | 30.000 |
| Std 7.5 | 7.5 | 2.250 | 27.75 | 30.000 |
| Std 5 | 5 | 1.500 | 28.50 | 30.000 |
| Std 2.5 | 2.5 | 0.750 | 29.25 | 30.000 |
| Std 1 | 1 | 0.299 | 29.70 | 30.000 |
| Std 0.5 | 0.5 | 0.149 | 29.85 | 30.000 |
| Std 0.25 | 0.25 | 0.074 | 29.93 | 30.000 |
| Std 0.1 | 0.1 | 0.029 | 29.97 | 30.000 |
| Std 0.05 | 0.05 | 0.014 | 29.99 | 30.000 |
| Std 0.0017 | 0.0017 | 0.000 | 30.00 | 30.000 |

A standard addition calibration dataset was prepared as shown in Table 14:

**Table 14**

| Standard used for addition | Pipetting different STD solution mL | Converted 100% ethanol (mL) | Total ethanol(mL) | Total volume in reactor (mL) | Final ethanol concentration (%) |
|---|---|---|---|---|---|
| Neat ethanol | 12.712 | 12.712 | 47.223 | 466.483 | 10.12 |
| Neat ethanol | 12.074 | 12.074 | 34.510 | 453.771 | 7.61 |
| Neat ethanol | 11.387 | 11.387 | 22.436 | 441.696 | 5.08 |
| Neat ethanol | 6.655 | 6.655 | 11.049 | 430.309 | 2.57 |
| Neat ethanol | 2.254 | 2.254 | 4.394 | 423.654 | 1.04 |
| 10% standard | 10.885 | 1.088 | 2.140 | 421.401 | 0.51 |
| 10% standard | 6.361 | 0.636 | 1.052 | 410.516 | 0.26 |
| 10% standard | 2.155 | 0.215 | 0.415 | 404.155 | 0.10 |
| 10% standard | 2.000 | 0.200 | 0.200 | 402.000 | 0.05 |

A third calibration dataset was built using offline ethanal analysis by headspace gas chromatography with flame ionization detection (HS-GC-FID). The method overview is shown in Table 15:

**Table 15.**

| Instrument Settings: | | | | |
|---|---|---|---|---|
| GC system: | Aglient GC System 7890A | | | |
| HS sampler: | Agilent Headspace Sampler 7697A | | | |
| GC column: | Agilent J&W HP-5ms, 30m, 0.25mm, 7 inch cage (19091S-433) | | | |

| GC parameters: | | | HS sampler parameters: | |
|---|---|---|---|---|
| Initial oven temperature | 50°C | | Equilibrium time: | 10 min. |
| Hold time | 1 min. | | Injection Duration: | 0.5 min. |
| Ramp | 10°C/min. | | Cycle time | 13 min. |
| Final oven temperature | 120°C | | Oven | 80°C |
| Carrier gas (He) flow | 1 mL/min. | | Loop | 110°C |
| Split ratio | 50:1 | | Transfer line | 140°C |
| Inlet heater | 180°C | | Transfer line | 0.32 mm |
| Inlet septum purge | 3 mL/min. | | | |
| Detector heater | 260°C | | | |
| Air flow | 450 mL/min. | | | |
| H₂ flow | 40 mL/min. | | | |
| Makeup flow | 40 mL/min. | | | |

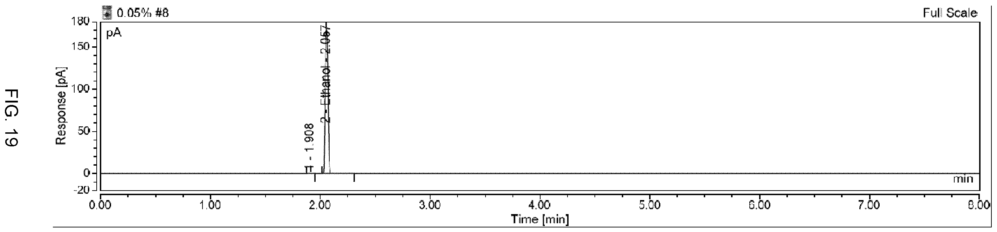

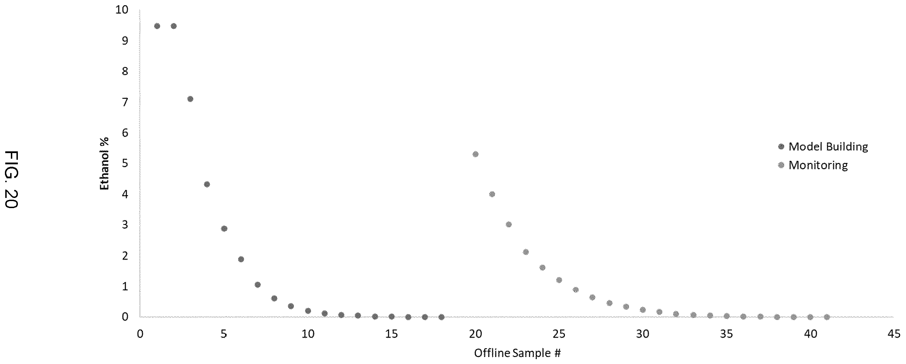

An exemplary chromatogram is shown in Fig. 19. Fig. 20 shows the offline results from the first and second TFF runs. The first TFF collected data for model building, 44 mL ethanol was added to feed tank to make ~10% starting solution. The second TFF used model built from standard set for monitoring, 22 mL ethanol was added to feed tank to make ~10% starting solution.

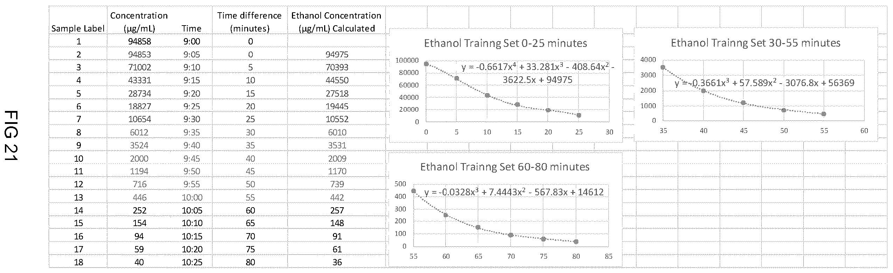

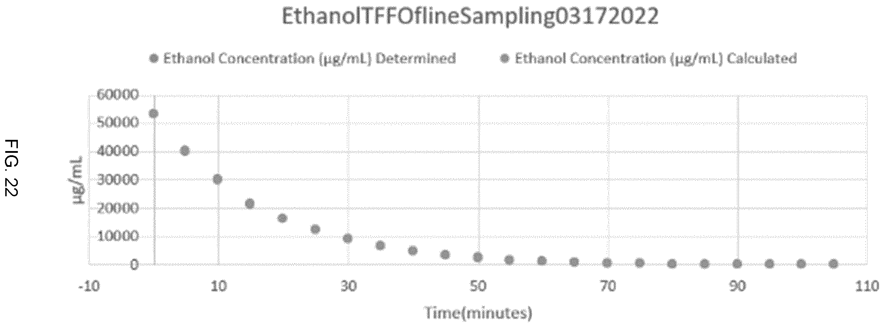

TFF-modeling building data set. Offline analysis was sampling at every 5 minutes. The ProCellics^{™} Raman analyzer probe sampled at every 2-3/4 minutes, polynomial regressions were used to predict the ethanol concentration at each time point Raman spectrum recorded. Due to the very broad dynamic range, 3 different polynomial regression models were applied to different sections to assure the prediction accuracy. The regression models aligning offline HS-GC-FID and Raman for ethanol clearance by TFF-modeling building data set are shown in Fig. 21. Fig. 22 shows alignment of offline HS-GC-FID and Raman for ethanol clearance by TFF-modeling building data set over 105 minutes.

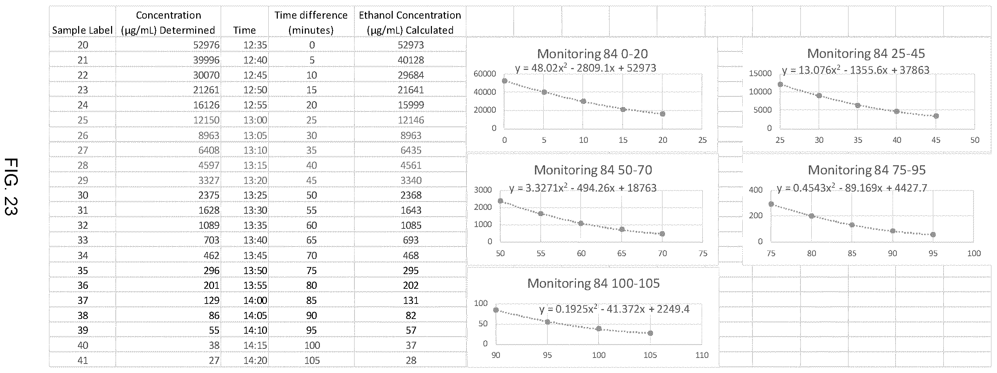

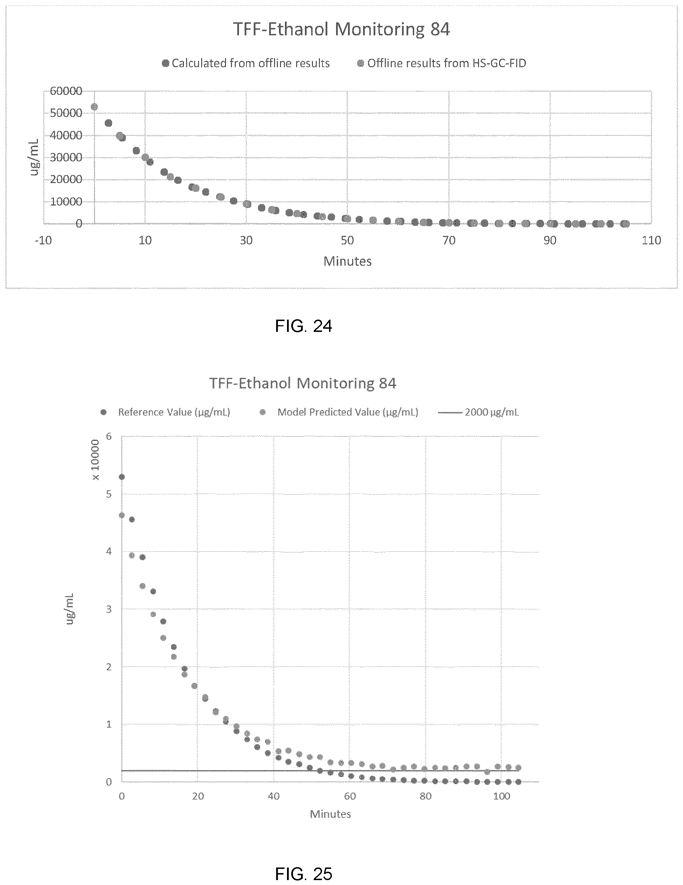

Next, the offline HS-GC-FID and Raman data for ethanol clearance by TFF-monitoring dataset were aligned. Polynomial regressions were used to predict the ethanol concentration at each time point Raman spectra were recorded. Due to the very broad dynamic range, five different polynomial regression models were applied to different sections to assure the prediction accuracy, as shown in Fig. 23. Fig. 24 shows the alignment of offline HS-GC-FID and polynomial regression models predicted results for each particular Raman data point for ethanol clearance by TFF-monitoring dataset.

As in example 1, models were built from the combined standard dataset and the offline analysis dataset, and then the model was optimized for low concentrations.

For the combined standard dataset, the individual standard dataset and standard addition dataset were used to build the model. For data processing parameters, SG (Savitzky-Golay) derivative and the selected Raman shift ranges:800-1710, 2650-3100 cm⁻¹ were used. The model, shown in FIG. 25, and the data is shown in Table 16 showed a large deviation from reference value, and had a detection limit around 2000 µg/mL.

**Table 16**

| TFF Time(minutes) | Reference Value (µg/mL) | Model Predicted Value (µg/mL) | Recovery (%) |
|---|---|---|---|
| 0.0 | 52973.0 | 46309.5 | 87 |
| 2.7 | 45638.1 | 39367.3 | 86 |
| 5.5 | 38997.8 | 34039.4 | 87 |
| 8.2 | 33084.5 | 29087.4 | 88 |
| 11.0 | 27897.7 | 25017.6 | 90 |
| 13.7 | 23437.6 | 21777.5 | 93 |
| 16.5 | 19704.4 | 18716.4 | 95 |
| 19.2 | 16697.9 | 16600.2 | 99 |
| 22.0 | 14418.0 | 14813.2 | 103 |
| 24.7 | 12324.7 | 12084.7 | 98 |
| 27.5 | 10474.7 | 10993.1 | 105 |
| 30.2 | 8822.8 | 9672.4 | 110 |
| 33.0 | 7368.7 | 8384.76 | 114 |
| 35.7 | 6112.6 | 7359.85 | 120 |
| 38.5 | 5054.3 | 6977.37 | 138 |
| 41.3 | 4193.9 | 5351.47 | 128 |
| 44.0 | 3531.4 | 5404.36 | 153 |
| 46.8 | 3066.8 | 4827.19 | 157 |
| 49.5 | 2448.8 | 4328.81 | 177 |
| 52.3 | 2020.5 | 4309.95 | 213 |
| 55.0 | 1642.5 | 3361.39 | 205 |
| 57.8 | 1314.9 | 3327.71 | 253 |
| 60.5 | 1037.7 | 3265.32 | 315 |
| 63.3 | 810.8 | 3085.6 | 381 |
| 66.0 | 634.2 | 2676.17 | 422 |
| 68.8 | 508.0 | 2841.57 | 559 |
| 71.5 | 432.2 | 2190.89 | 507 |
| 74.3 | 311.2 | 2481.38 | 797 |
| 77.0 | 255.0 | 2662.56 | 1044 |
| 79.8 | 205.6 | 2268.1 | 1103 |
| 82.5 | 163.1 | 2513.18 | 1541 |
| 85.3 | 127.5 | 2377.5 | 1864 |
| 88.0 | 98.8 | 2510.66 | 2542 |
| 90.8 | 76.9 | 2637.52 | 3429 |
| 93.5 | 61.9 | 2674.47 | 4318 |
| 96.3 | 53.8 | 1804.62 | 3353 |
| 99.0 | 40.2 | 2664.51 | 6627 |
| 101.8 | 32.7 | 2568.2 | 7849 |
| 104.5 | 28.2 | 2476.89 | 8799 |

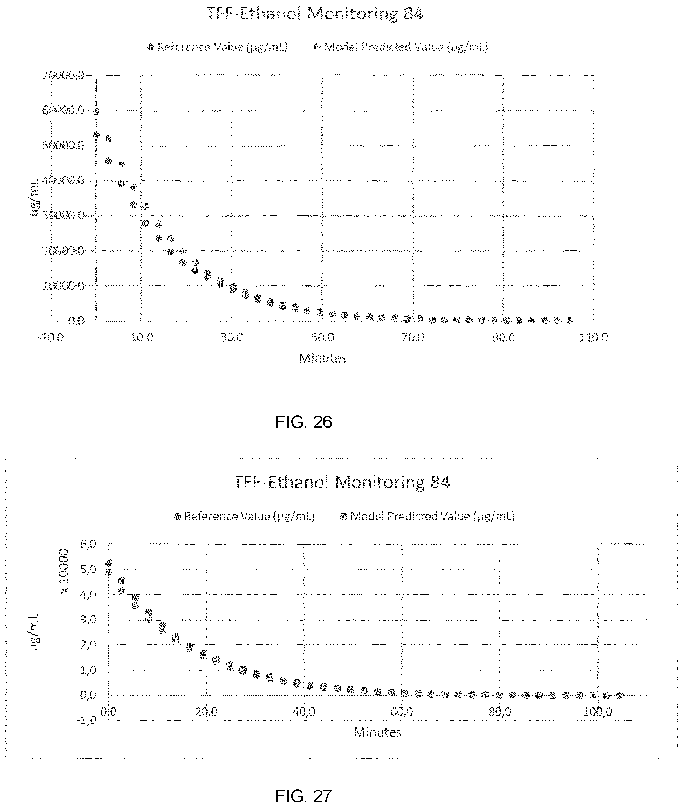

The offline sample analyses dataset was used to build a second model. The same data processing parameters were used. The model, shown in FIG. 26, and the data is shown in Table 17, was very comparable from reference value and had a significantly improved detection limit, however, the prediction accuracy below 1800 µg/mL is poor.

**Table 17**

| TFF Time(minutes) | Reference Value (µg/mL) | Model Predicted Value (µg/mL) | Recovery (%) |
|---|---|---|---|
| 0.0 | 52973.0 | 61361.9 | 113 |
| 2.7 | 45638.1 | 59645.4 | 113 |
| 5.5 | 38997.8 | 51793.5 | 115 |
| 8.2 | 33084.5 | 44775.2 | 116 |
| 11.0 | 27897.7 | 38288.8 | 117 |
| 13.7 | 23437.6 | 32717.1 | 118 |
| 16.5 | 19704.4 | 27612 | 119 |
| 19.2 | 16697.9 | 23363.7 | 118 |
| 22.0 | 14418.0 | 19734.6 | 115 |
| 24.7 | 12324.7 | 16581.7 | 113 |
| 27.5 | 10474.7 | 13909.8 | 111 |
| 30.2 | 8822.8 | 11631.6 | 111 |
| 33.0 | 7368.7 | 9765.38 | 111 |
| 35.7 | 6112.6 | 8150.5 | 111 |
| 38.5 | 5054.3 | 6762.58 | 112 |
| 41.3 | 4193.9 | 5656.45 | 112 |
| 44.0 | 3531.4 | 4677.8 | 111 |
| 46.8 | 3066.8 | 3903.79 | 104 |
| 49.5 | 2448.8 | 3202.79 | 108 |
| 52.3 | 2020.5 | 2652.38 | 111 |
| 55.0 | 1642.5 | 2233.39 | 110 |
| 57.8 | 1314.9 | 1807.59 | 112 |
| 60.5 | 1037.7 | 1479.09 | 122 |
| 63.3 | 810.8 | 1263.55 | 126 |
| 66.0 | 634.2 | 1021.88 | 131 |
| 68.8 | 508.0 | 833.959 | 135 |
| 71.5 | 432.2 | 685.732 | 141 |
| 74.3 | 311.2 | 609.197 | 161 |
| 77.0 | 255.0 | 501.996 | 192 |
| 79.8 | 205.6 | 490.188 | 181 |
| 82.5 | 163.1 | 372.299 | 222 |
| 85.3 | 127.5 | 362.848 | 290 |
| 88.0 | 98.8 | 369.287 | 247 |
| 90.8 | 76.9 | 244.182 | 273 |
| 93.5 | 61.9 | 210.168 | 491 |
| 96.3 | 53.8 | 304.002 | 435 |
| 99.0 | 40.2 | 234.311 | 487 |
| 101.8 | 32.7 | 195.861 | 627 |
| 104.5 | 28.2 | 205.006 | 781 |

Another model was built using offline data, but only including data in the range from 1 to 1000 µg/mL in the offline samples analysis dataset. The calibration curve is shown in FIG. 27, and the data is shown in Table 18. This model worked significantly better than model built from the complete training dataset, with high prediction accuracy even in the high concentration range. 100 ppm and below cam be predicted with good accuracy. The limitation of the model is approximately 35 ppm. Example 2 shows the methods herein are also suitable for the monitoring of solvent removal by TFF or other filtration processes in LNP or liposome production.

**Table 18**

| TFF Time (minutes) | Reference Value (µg/mL) | Model Predicted Value (µg/mL) | Recovery (%) |
|---|---|---|---|
| 0.0 | 66087.0 | 50680.900 | 93 |
| 2.0 | 54696.9 | 49070.400 | 91 |
| 4.7 | 42257.4 | 41664.400 | 91 |
| 7.5 | 33225.8 | 35667.400 | 92 |
| 10.2 | 26742.5 | 30330.800 | 93 |
| 13.0 | 21948.0 | 25938.400 | 94 |
| 15.7 | 17982.6 | 22068.600 | 95 |
| 18.5 | 13986.9 | 18815.400 | 96 |
| 21.2 | 9101.3 | 16028.700 | 94 |
| 24.0 | 7189.0 | 13583.000 | 93 |
| 26.7 | 5728.7 | 11514.800 | 93 |
| 29.5 | 4502.7 | 9698.450 | 93 |
| 32.2 | 3492.9 | 8202.530 | 93 |
| 35.0 | 2681.3 | 6858.860 | 95 |
| 37.7 | 2049.8 | 5817.910 | 96 |
| 40.5 | 1580.3 | 4829.970 | 96 |
| 43.2 | 1254.9 | 4020.230 | 94 |
| 46.0 | 1055.4 | 3321.170 | 89 |
| 48.7 | 785.2 | 2722.480 | 91 |
| 51.5 | 605.3 | 2232.070 | 91 |
| 54.2 | 468.3 | 1847.710 | 89 |
| 57.0 | 366.5 | 1459.380 | 90 |
| 59.8 | 292.4 | 1188.160 | 94 |
| 62.5 | 238.5 | 971.441 | 92 |
| 65.3 | 197.1 | 746.145 | 100 |
| 68.0 | 160.8 | 633.390 | 90 |
| 70.8 | 122.0 | 458.774 | 83 |
| 73.5 | 91.8 | 356.735 | 92 |
| 76.3 | 78.0 | 287.249 | 97 |
| 79.0 | 65.7 | 247.696 | 80 |
| 81.8 | 55.1 | 163.987 | 98 |
| 84.5 | 45.9 | 160.185 | 99 |
| 87.3 | 38.3 | 126.412 | 42 |
| 90.0 | 32.3 | 41.300 | 76 |
| 92.8 | 27.8 | 58.201 | 101 |
| 95.5 | 24.9 | 62.844 | 73 |
| 98.3 | 20.6 | 39.516 | 49 |
| 101.0 | 18.5 | 19.663 | -64 |
| 103.8 | 16.6 | -20.950 | -36 |
| 106.5 | 14.9 | -10.253 | 146 |
| 109.3 | 13.4 | 44.4 | 331 |

The examples herein are for illustrative purposes only and are not meant to limit the scope of the invention as defined by the claims.

## Claims

1. A method for monitoring solvent clearance in a downstream process comprising the steps:
a. providing a calibration model for quantitating concentration of the solvent
b. collecting Raman spectra during the solvent clearance process
c. using the calibration model to quantitate the concentration of solvent.

2. The method of claim 1 wherein the monitoring of solvent clearance is done in real-time.

3. The method of either of claims 1 or 2 wherein the Raman spectra are collected in situ.

4. The method of any of claims 1 -3 wherein steps (b) and (c) are repeated until the concentration of solvent is below a pre-defined level.

5. The method of claim 4 wherein the pre-defined level of solvent concentration corresponds to the international council for harmonisation (ICH) guideline for residual solvents Q3C(R8) for that solvent.

6. The method of any of claims 1 - 5 wherein the calibration model is selected from the group consisting of a linear regression model, a principal components regression (PCR) model, a partial least squares (PLS) regression model, a support vector machines (SCM) model, a neural network model, and an artificial intelligence (AI) model, and is preferably a PLS regression model.

7. The method of any of claims 1 - 6 wherein the downstream process is a tangential flow filtration (TFF) process or a column purification process.

8. The method of any of claims 1 - 7 wherein the downstream process is a downstream antibody-drug conjugate (ADC) production process.

9. The method of claim 8 wherein the solvent is selected from the group consisting of DMSO, dimethylacetamide (DMA), isopropyl alcohol (IPA) and combinations thereof, and is preferably DMSO.

10. The method of any of claims 1 - 7 wherein the downstream process is a downstream lipid nanoparticle (LNP) or liposome production process.

11. The method of claim 10 wherein the solvent is ethanol.

12. An inline, real-time method for monitoring solvent clearance from a filter retentate in a downstream filtration process comprising the steps of
a. providing a regression model for quantitating concentration of the solvent
b. collecting Raman spectra of the filtration retentate in situ
c. using the regression model to quantitate the concentration of solvent in the filtrate retentate in real time
d. continuously monitoring solvent clearance by repeating steps b and c until the concentration of solvent in the filtrate retentate is below a predefined level.

13. The method of claim 12 wherein the filtration process is a TFF process.

14. The method of either of claims 12 or 13 wherein the regression model is selected from a linear regression model, a principal components regression (PCR) model, a partial least squares (PLS) regression model, a support vector machines (SCM) model, a neural network model, or an artificial intelligence (AI) model, preferably a PLS regression model.

15. The method of any of claims 12 - 14, wherein the predetermined level of solvent concentration corresponds to the international council for harmonisation (ICH) guideline for residual solvents Q3C(R8) for that solvent.

16. A method for monitoring trace solvent concentration in a filtration retentate in a downstream process comprising the steps of:
a. providing a calibration model for determining concentration of the solvent
b. collecting Raman spectra during the solvent removal process
c. using the calibration model to quantitate the concentration of solvent.

17. The method of claim 16 wherein the downstream process is a tangential flow filtration (TFF) process.

18. The method of either of claims 16 or 17 wherein the calibration model is selected from a linear regression model, a principal components regression (PCR) model, a partial least squares (PLS) regression model, a support vector machines (SCM) model, a neural network model, or an artificial intelligence (AI) model.

19. The method of any of claims 16 - 18 wherein the monitoring is done in real-time.

20. The method of any of claims 16 - 19 wherein the monitoring is done in situ.

21. The method of any of claims 16 - 20 wherein steps b and c are repeated until the trace solvent concentration is below a pre-defined level.

22. The method of claim 21 wherein the pre-defined level of trace solvent concentration corresponds to the international council for Harmonisation (ICH) Guideline for Residual Solvents Q3C(R8) for that solvent.

23. The method of any of claims 16 - 22 wherein the downstream process is a downstream antibody-drug conjugate (ADC) production process.

24. The method of claim 23 wherein the solvent is selected from the group consisting of DMSO, DMA, IPA and combinations thereof.

25. The method of any of claims 16 - 22 wherein the downstream process is a downstream lipid nanoparticle (LNP) or liposome production process.

26. The method of claim 25 wherein the solvent is ethanol.

27. A method for quantitating residual solvent in a downstream process comprising the steps:
a. providing a calibration model for quantitating concentration of the solvent
b. collecting Raman spectra of a filter retentate during the downstream process
c. using the calibration model to quantitate the concentration of residual solvent in the filter retentate.

28. The method of claim 27 wherein the calibration model is selected from a linear regression model, a principal components regression (PCR) model, a partial least squares (PLS) regression model, a support vector machines (SCM) model, a neural network model, or an artificial intelligence (AI) model, and is preferably a PLS regression model.

29. The method either of claims 27 or 28 wherein the downstream process is a tangential flow filtration (TFF) process or a column purification process.

30. The method of any of claims 27 - 29 wherein the quantitation of residual solvent is done in real-time.

31. The method of any of claims 27 - 30 wherein the Raman spectra are collected in situ.

32. The method of any of claims 27 - 31 wherein steps b and c are repeated until the residual solvent concentration is below a pre-defined level.

33. The method of any of claims 27 - 32 wherein the pre-defined level of residual solvent corresponds to the international council for harmonisation (ICH) guideline for residual solvents Q3C(R8) for that solvent.

34. The method of any of claims 27 - 33 wherein the downstream process is a downstream antibody-drug conjugate (ADC) production process.

35. The method of claim 34 wherein the solvent is selected from the group consisting of DMSO, DMA, IPA and combinations thereof.

36. The method of any of claims 27 - 33 wherein the downstream process is a downstream lipid nanoparticle (LNP) of liposome production process.

37. The method of claim 36 wherein the solvent is ethanol.

38. A method for measuring solvent clearance in a downstream process comprising the steps:
a. providing a calibration model for quantitating concentration of the solvent
b. collecting Raman spectra during the solvent clearance process
c. using the calibration model to quantitate the concentration of solvent.

39. The method of claim 35 wherein the calibration model is selected from a linear regression model, a principal components regression (PCR) model, a partial least squares (PLS) regression model, a support vector machines (SCM) model, a neural network model, or an artificial intelligence (AI) model.

40. The method of either of claims 38 or 39 wherein the downstream process is TFF or column chromatography.
